# EUROPEAN PATENT SPECIFICATION

(11) **EP 3 895 633 B1**
(45) Date of publication and mention of the grant of the patent: **21.05.2025**
(21) Application number: 21163947.1
(22) Date of filing: 22.03.2021
(51) Int. Cl.: A61B 17/221, A61B 17/22, A61B 17/00

(54) **WIRE-EQUIPPED STENT**
DRAHTBESTÜCKTER STENT
STENT ÉQUIPÉ D'UN FIL

(30) Priority: 24.03.2020 JP 2020052300
(43) Date of publication of application: 20.10.2021
(73) Proprietor: Otsuka Medical Devices Co., Ltd., Tokyo 101-8535 (JP)
(72) Inventor: Doi, Yuta, Tokyo, 103-0023 (JP)
(74) Representative: Taruttis, Tilman

(56) References cited:
- WO-A1-2014/139845
- WO-A1-2020/039082
- JP-A- 2016 185 206

## Description

### BACKGROUND OF THE INVENTION

### Field of the Invention

The present invention relates to a wire-equipped stent.

### Related Art

In the related art, a catheter-stent system (hereinafter also referred to as a "CS system") including a wire and a stent connected to the distal end of the wire is used to capture a blood clot formed in a blood vessel. When such a CS system is used, the stent is inserted into a blood vessel to capture a blood clot, and then the wire is pulled through the catheter so that the blood clot is retrieved with the stent pulled out of the body. A known catheter used for such a CS system has a structure including two types of coils overlapping each other (see, for example, Patent Document 1).

Patent Document 1: Japanese Unexamined Patent Application, Publication No. 2016-185206
Further prior art document WO 2014/139845 A1 relates to a clot retrieval device for removing occlusive clot from a blood vessel. A shaft has a coil adjacent its distal end. Adjacent to the coil is a sleeve on the shaft. The sleeve and shaft may be coated with a material to reduce friction and thrombogenicity. WO 2020/039082A1 also discloses a clot retrieval device.

JP 2016 185206A relates to a catheter having an internal layer, an external layer arranged on the outer surface side of the internal layer, and a reinforcement body sandwiched between the internal layer and the external layer. The reinforcement body has a braided part formed tubularly by two or more wires, and a coil part fixed to the braided part in the elastically deformed state from a natural state.

### SUMMARY OF THE INVENTION

The invention is as defined in claim **1.** Preferred embodiments of the invention are defined in the dependent claims.
A catheter as described in the related art has a highly-flexible, distal end portion and also has a high ability to transmit torque to the distal end portion. In such a catheter having the two coils overlapping each other, however, a resin covering the two coils has an undulated inner surface due to the steps formed by the coils. A wire-equipped stent to be inserted into such a catheter includes a wire and a coiled member connected to the distal end of the wire. The coiled member is a spirally coiled metal wire and thus has an undulated outer circumference.

When such a conventional wire-equipped stent is inserted into the above catheter and used, the undulated inner surface of the catheter rubs against the undulated circumference of the coiled member connected to the wire, which increases the sliding resistance of the wire-equipped stent in the catheter. As the sliding resistance of the wire-equipped stent increases inside the catheter, the ease of handling for the operator who pushes and pulls the wire decreases. Therefore, a need exists to improve the ease of handling of a wire-equipped stent inserted in a catheter having an undulated inner surface.

It is an object of the present invention to provide a wire-equipped stent, a catheter, and a catheter-stent system each having further improved ease of handling.

A first aspect of the present invention relates to a wire-equipped stent including: a wire; a stent connected to a distal end portion of the wire; and a highly smooth portion provided at or adjacent to a distal portion of the wire, the wire-equipped stent being adapted for insertion into and use in a catheter having a structure including a mesh coil and a spiral coil overlapping each other.

The highly smooth portion may be free of a coiled member.

The highly smooth portion may have an outer surface with a surface roughness Ra of 0.5 nm or more and less than 2.0 nm.

The highly smooth portion may be a solid layer including a hydrophobic material.

The highly smooth portion may be a fluid layer including a hydrophilic material.

The stent may be adapted to be delivered together with the catheter to a region at or downstream of M1 region in a skull and to be used together with the catheter in the skull.

The stent may also be adapted for use to retrieve a blood clot formed in a cerebral blood vessel in a region at or downstream of the M1 region.

A second aspect disclosed herein relates to a catheter including: a structure including a mesh coil and a spiral coil overlapping each other, the catheter being adapted to receive inside and be used together with a wire-equipped stent including: a wire having a distal portion provided with a highly smooth portion free of a coiled member; and a stent connected to a distal end portion of the wire.

The catheter may have an undulated inner surface.

The catheter may be adapted to be delivered to a region at or downstream of M1 region in a skull and to be used in the skull.

A third aspect disclosed herein relates to a catheter-stent system including the wire-equipped stent and the catheter.

The present invention makes it possible to provide a wire-equipped stent, a catheter, and a catheter-stent system each having further improved ease of handling.

### BRIEF DESCRIPTION OF THE DRAWINGS

FIG. 1 is a view showing the structure of a wire-equipped stent 10;
FIG. 2 is a schematic view showing the structure of a catheter-stent system 1;
FIG. 3 is a schematic view showing the structure of a mesh coil 211 and a spiral coil 212 in a catheter 20; and
FIGS. 4A to 4E are schematic views showing a procedure for removing a blood clot from a blood vessel using a catheter-stent system 1.

### DETAILED DESCRIPTION OF THE INVENTION

Hereinafter, a wire-equipped stent according to the invention, a catheter, and a catheter-stent system will be described. It should be noted that the drawings attached hereto are all schematic views in which, for ease of understanding, the shape, scale size, aspect ratio, or other features of each component are changed or exaggerated relative to the actual component. It should also be noted that hatching for indicating a cross-section of some of components is omitted for clarity from the drawings.

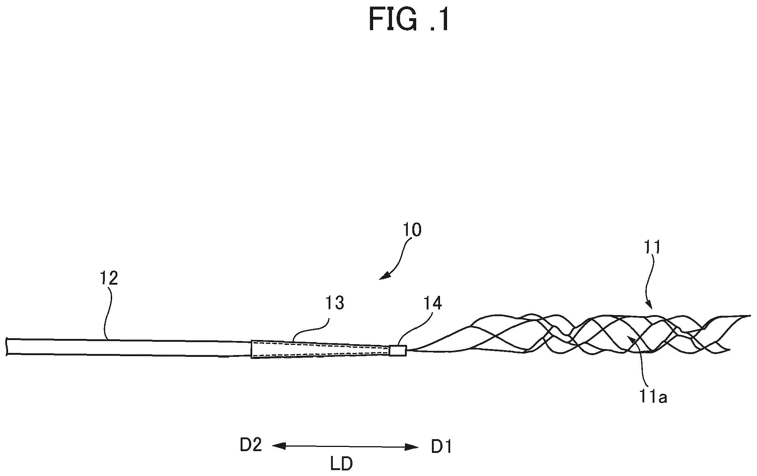

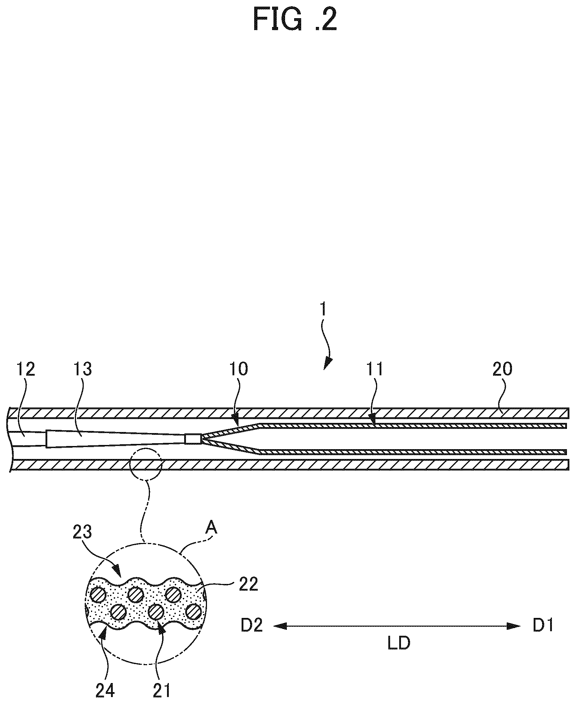

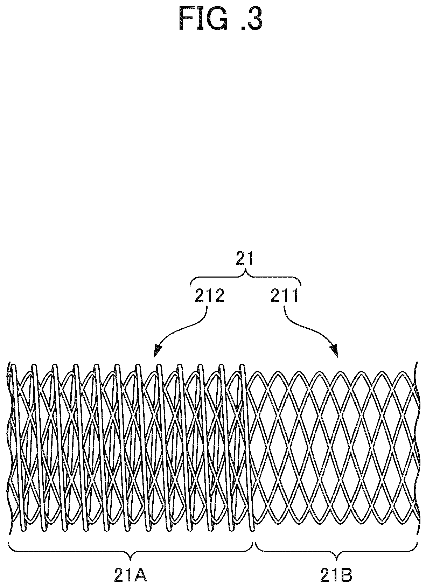

FIG. 1 is a view showing the structure of a wire-equipped stent 10. FIG. 2 is a schematic view showing the structure of a catheter-stent system 1. FIG. 3 is a schematic view showing the structure of a mesh coil 211 and a spiral coil 212 in a catheter 20. As used herein, the term "distal side D1" refers to a side away from the operator in axis direction LD of the catheter-stent system (CS system) 1 described herein, and the term "proximal side D2" refers to a side close to the operator in the axis direction LD.

The CS system 1 according to an embodiment of the present invention includes a wire-equipped stent 10 and a catheter 20, which will be described later. As shown in FIG. 1, the wire-equipped stent 10 includes, as main components, a stent 11, a pusher wire 12, and a cover layer (highly smooth portion) 13.

The stent 11 is a part configured to capture a blood clot. The stent 11 is substantially cylindrical. The circumferential wall of the stent 11 includes a plurality of closed cells 11a that are each made of a wire-shaped material surrounding the cell. As used herein, the term "the circumferential wall of the stent 11" means a portion that separates the inside of the substantially cylindrical structure of the stent 11 from the outside. As used herein, the term "closed cell" refers to an opening portion surrounded by a wire-shaped material, which forms the mesh patterns of the stent 11 and is also called an opening or a compartment. It should be noted that FIG. 1 shows the unloaded state of the stent 11. The term "unloaded state" refers to the state of the stent 11 not radially compressed.

The stent 11 with the structure described above may be formed by laser-processing a tube including, for example, a biocompatible material, in particular preferably a tube made of a superelastic alloy. If the stent 11 should be made at a reduced cost from a superelastic alloy tube, the stent 11 is preferably formed by subjecting a superelastic alloy tube with a diameter of about 2 to 3 mm to laser processing and then expanding the tube to a desired diameter to subject the tube to shape-memory treatment. It will be understood that the stent 11 may be formed not only by laser processing but also by other methods such as cutting work or knitting a wire-shaped metal material into a tube.

The stent is preferably made of a highly rigid, biocompatible material. Such a material may be, for example, titanium, nickel, stainless steel, platinum, gold, silver, copper, iron, chromium, cobalt, aluminum, molybdenum, manganese, tantalum, tungsten, niobium, magnesium, calcium, or an alloy containing any of the above. Such a material may also be, for example, a synthetic resin material, such as a polyolefin such as polyethylene (PE) or polypropylene (PP), or polyamide, polyvinyl chloride, polyphenylene sulfide, polycarbonate, polyether, or polymethyl methacrylate. Such a material may also be, for example, a biodegradable resin (biodegradable polymer), such as polylactic acid (PLA), polyhydroxybutyrate (PHB), polyglycolic acid (PGA), or poly-ε-caprolactone.

Among these materials, titanium, nickel, stainless steel, platinum, gold, silver, copper, and magnesium or alloys containing any of them are preferred. Examples of such alloys include Ni-Ti alloys, Cu-Mn alloys, Cu-Cd alloys, Co-Cr alloys, Cu-Al-Mn alloys, Au-Cd-Ag alloys, and Ti-Al-V alloys. Examples of such alloys also include alloys of magnesium and Zr, Y, Ti, Ta, Nd, Nb, Zn, Ca, Al, Li, Mn, or the like. Among these alloys, Ni-Ti alloys are preferred.

The stent 11 has a proximal side D2 end portion connected to a marker 14. The marker 14 is a member that serves as a maker to identify the position of the proximal side D2 end portion of the stent 11. A pusher wire 12 is connected to the proximal side D2 end of the marker 14. The pusher wire 12 is a member that is to be handled by the operator who moves the stent 11. To move the stent 11 in a catheter 20 (describe later) or a blood vessel, the operator pushes or pulls the pusher wire 12 through a handling portion (not shown) connected to a proximal side D2 portion of the pusher wire 12.

A cover layer 13 is provided on a distal side D1 portion of the pusher wire 12. The cover layer 13 is a layer provided to increase the smoothness for contact with the inner surface of the catheter 20 (described later). The cover layer 13 is provided to cover the outer surface of a distal side D1 portion of the pusher wire 12. The distal side D1 portion of the pusher wire 12 may be, for example, a portion over a length of 50 mm to 3,000 mm from the distal side D1 end of the pusher wire 12 to a proximal side D2 site. The distal side D1 end of the cover layer 13 is bonded to the marker 14 with an adhesive (not shown) in between them. Preferably, the distal side D1 end of the cover layer 13 is bonded to the marker 14 with no gap between them as shown in FIG. **1****.** However, a gap of few mm may remain between the distal side D1 end and the marker 14.

The cover layer 13 may include a solid layer of a hydrophobic resin. Such a solid hydrophobic resin layer may be made of, for example, polytetrafluoroethylene (PTFE) or diamond-like carbon (DLC). These materials can form a surface with low friction resistance (highly smooth surface) to reduce the sliding resistance generated when the pusher wire 12 rubs against the undulated inner surface of the catheter 20. As long as the cover layer 13 is provided on a distal side D1 portion of the pusher wire 12, the cover layer 13 may have a length in the axis direction LD larger or smaller than the length of the distal side D1 portion.

Alternatively, the cover layer 13 may include a fluid layer of a hydrophilic resin. Such a hydrophilic fluid layer may be made of, for example, hyaluronic acid, an MPC polymer, or the like. These materials may be applied to an outer surface of the pusher wire 12 to form a semisolid layer having a surface with lower friction resistance (highly smooth surface). Such a layer reduces the sliding resistance generated when the pusher wire 12 rubs against the undulated inner surface of the catheter 20.

In the wire-equipped stent 10 according to an embodiment of the present invention, the highly smooth portion may include a material having an outer surface with a surface roughness (arithmetic average roughness) Ra of 0.5 nm or more and less than 2.0 nm. In this embodiment, the highly smooth portion may include any material having human-safe properties. The wire-equipped stent 10 according to an embodiment of the present invention may include a coiled member between the distal side D1 end of the pusher wire 12 and the stent 11. In such a case, a material satisfying the above surface roughness Ra conditions may be provided on the outer surface of the coiled member, so that coiled member can serve as a highly smooth portion. In other words, the wire-equipped stent according to the present invention may include or may not include a coiled member as long as it has the highly smooth portion provided at or adjacent to a distal portion of the wire. The wire-equipped stent 10 according to an embodiment of the present invention may not include the cover layer 13. In such an embodiment, a distal side D1 portion of the pusher wire 12 serves as the highly smoothy portion. In the wire-equipped stent 10 according to such an embodiment, the highly smooth portion does not include any coiled member.

As shown in FIG. 2, the CS system 1 according to an embodiment of the present invention includes the stent 11 configured as described above and a catheter 20. While being compressed radially, the stent 11 is inserted into the catheter 20. FIG. 2 schematically shows a cross-section of the radially compressed stent 11. In the CS system 1 shown in FIG. 2, the pusher wire 12 is pushed toward the distal side D1 to feed the stent 11 to a target site in a blood vessel, and pulled toward the proximal side D2 to remove the stent 11 from the body.

As shown in an enlarged area A in FIG. 2, the catheter 20 includes a core material portion 21 and a resin layer 22 covering the core material portion 21. The core material portion 21 includes a structure including a mesh coil 211 and a spiral coil 212 overlapping each other, which will be described later. Thus, the inner surface 23 and outer surface 24 of the catheter 20 are undulated due to the steps formed by the two coils.

As shown in FIG. 3, the core material portion 21 includes a mesh coil 211 and a spiral coil 212. The mesh coil 211 is a part including a metal wire knitted in the form of a mesh. The mesh coil 211 extends over the entire length of the catheter 20 in the axis direction LD. The spiral coil 212 is a part including a metal wire spirally wound on the outer circumference of the mesh coil 211.

As shown in Fig. 3, the catheter 20 according to this embodiment includes an overlapping portion 21A including a portion of the mesh coil 211 and the spiral coil 212; and a mesh portion 21B including only another portion of the mesh coil 211. The mesh portion 21B is mainly provided in a distal side D1 portion of the catheter 20. In the catheter 20, the distal side D1 portion including only a portion of the mesh coil 211 makes more flexible the distal end portion (distal side D1 portion) of the catheter 20. In the catheter 20, however, the overlapping portion 21A may extend over the entire length in the axis direction LD.

The catheter 20 configured as described above has an outer diameter of, for example, 0.55 mm to 2.10 mm. Its inner diameter is, for example, 0.33 mm to 1.78 mm. In the portion where the mesh coil 211 and the spiral coil 212 overlap, the resin layer has an inner surface with a surface roughness (arithmetic average roughness) Ra of, for example, about 1 µm to about 50 µm.

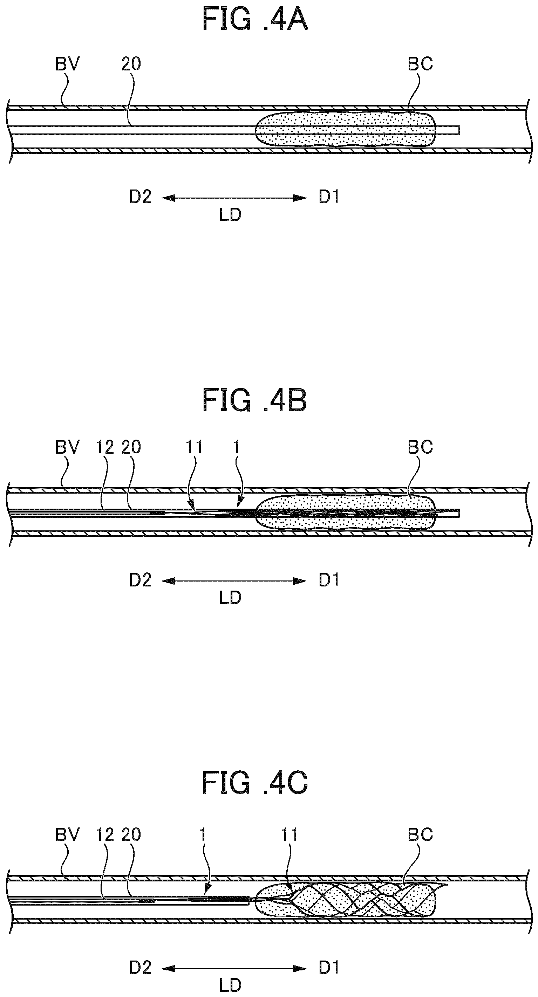

Next, an example of a method of using the catheter-stent system 1 equipped with the stent 11 according to the embodiment will be described. FIGS. 4A to 4E are schematic views showing a procedure for removing a blood clot from a blood vessel using the catheter-stent system **1.** In FIGS. 4A to 4E, solid lines are used to indicate the stent 11 and the pusher wire 12 in the catheter 20 so that it can be easily understood how the stent 11 deploys.

First, as shown in FIG. 4A, the catheter 20 is inserted into a patient's blood vessel BV to reach the position of a blood clot BC at a lesion site. The catheter 20 is fed until its distal end reaches a side D1 distal to the blood clot BC. In this regard, the catheter 20 may not penetrate the blood clot BC, and, for example, may be inserted between the blood clot BC and the inner surface of the blood vessel BV. Subsequently, the stent 11, being compressed radially, is inserted into the catheter 20. The stent 11, which has improved smoothness, can be pushed in with a force smaller than that for the conventional stent, even when inserted into a thinner catheter.

Subsequently, as shown in FIG. 4B, the pusher wire 12 is manipulated to push the radially compressed stent 11 along the lumen of the catheter 20. The distal end of the stent 11 is then positioned at a side D1 distal to the blood clot BC.

Subsequently, as shown in FIG. 4C, the stent 11 is pushed out of the distal end of the catheter 20 at the position of the blood clot BC and allowed to deploy. Specifically, the catheter 20 is pulled toward the proximal side D2 from the position shown in FIG. 4B so that the stent 11 is pushed out relative to the catheter 20 and deploys. The stent 11 is entirely pushed out of the catheter 20.

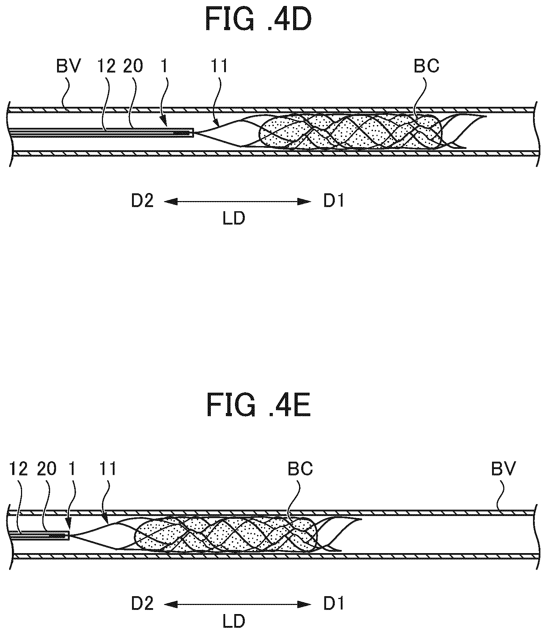

Subsequently, as shown in FIG. 4D, the pusher wire 12 and the catheter 20 are pulled toward the proximal side D2. As a result, as shown in FIG. 4E, the stent 11, which captures the blood clot BC, is successfully pulled out of the body. The blood clot BC is successfully removed from the body using the CS system 1 according to the procedure described above. It should be noted that FIGS. 4A to 4E show the outline of the operation for retrieving the blood clot BC and the actual operation for retrieving the blood clot BC from the body may include a variety of procedures depending on the lesion site and the like.

In the CS system 1 according to this embodiment, the highly smooth cover layer 13 between the marker 14 and the distal side D1 end of the pusher wire 12 successfully reduces the sliding resistance generated when the pusher wire 12 rubs against the undulated inner surface of the catheter 20. Therefore, the operator can use a smaller force to push or pull the pusher wire 12, which further improves the ease of handling of the CS system **1.**

The CS system 1 according to this embodiment may be mainly used to retrieve a formed blood clot from a cerebral blood vessel particularly in a region at or downstream of the M1 region inside the bone of the skull, in which the blood vessels have relatively small inner diameters and have many bent portions. In such a region, the conventional wire-equipped stent may have higher sliding resistance and lower ease of handling in the catheter used, which has a smaller inner diameter. In contrast, the CS system 1 according to this embodiment has higher ease of handling also in the area at or downstream of the M1 region inside the bone of the skull, because the wire-equipped stent has lower sliding resistance in the catheter.

Next, the results of testing on the sliding property of the wire-equipped stent according to the present invention will be described. Three types of wire-equipped stents having high sliding properties were prepared as Examples 1 to 3. The wire-equipped stent of Example 1 includes the pusher wire 12; and a highly smooth portion made of a PTFE layer (solid layer) with a thickness of 0.05 mm and a length of 250 mm on the outer surface of a distal side D1 portion of the pusher wire 12. The wire-equipped stent of Example 2 includes the pusher wire 12; and a highly smooth portion made of a hyaluronic acid resin layer (fluid layer) with a thickness of 0.001 mm and a length of 250 mm on the outer surface of a distal side D1 portion of the pusher wire 12. The wire-equipped stent of Example 3 includes a pusher wire and a stent that is connected to a distal side D1 end portion of the pusher wire with no coiled member between them. In the wire-equipped stent of Example 3, a distal side D1 portion of the pusher wire serves as a highly smooth portion. A wire-equipped stent prepared as a conventional example includes a pusher wire, a stent, and a coiled member between a distal side D1 end portion of the pusher wire and the stent. The conventional example is a general ready-made product used widely. The four types of wire-equipped stents were measured for tensile load under the conditions shown below.

Devices used:
a catheter
which includes a core material portion including a mesh coil and a spiral coil overlapping each other; and a resin layer having an inner surface with a surface roughness (arithmetic average roughness) Ra of 1.5 nm;
a digital force gauge (push pull gauge);
a pulling device;
a thermostatic chamber; and
a thermometer

### Test conditions:

Speed: 60 mm/min
Pulling distance: the effective length of the stent + 10 mm
Test temperature: 37°C ± 2°C

Test method:
(1) confirming with the thermometer that the thermostatic chamber has a temperature of 37°C ± 2°C;
(2) placing the catheter in the thermostatic chamber,
   in which the catheter is in an S shape having an arc with a diameter of 5 mm for simulation of actual use through a bent portion in the body;
(3) inserting the wire-equipped stent into the catheter from the proximal side until the distal end of the stent is positioned at a site distal to the S-curve portion of the catheter;
(4) connecting the proximal end of the wire-equipped stent to the digital force gauge disposed in the pulling device;
(5) pulling the wire-quipped stent constantly at the specified speed toward the proximal side using the pulling device while the catheter and the wire-equipped stent are kept in an S shape; and
(6) measuring tensile loads using the digital force gauge when the wire-equipped stent is pulled over a distance equal to the effective length + 10 mm and recording the minimum and maximum values of the tensile load.

The test on each of the wire-equipped stents of Examples 1 to 3 and the comparative example was repeated multiple times to measure tensile loads. An average tensile load of less than 1 N was evaluated as good (∘), 1 N or more and less than 3 N as fair (Δ), and 3 N or more as poor (×). Among them, the rates evaluated as poor (×) are not acceptable, whereas the rates evaluated as fair (Δ) and good (o) are acceptable. The term "fair" (Δ) means a rating level acceptable practically but slightly lower than the level "good" (o).

Table 1 shows the results of the evaluation of each of the wire-equipped stents of Examples 1 to 3 and the comparative example by the test.

**[Table 1]**

| | Example 1 | Example 2 | Example 3 | Comparative Example |
|---|---|---|---|---|
| Tensile Load (N) | 0.7 | 0.5 | 0.9 | 3.2 |
| Evaluations | ○ | ○ | ○ | × |

As shown in Table 1, the wire-equipped stents of Examples 1 to 3 all had an average tensile load of less than 1 N and were evaluated as good (o). The wire-equipped stent of the comparative example had an average tensile load of at least 3 N and was evaluated as poor (×). The results demonstrate that the wire-equipped stents of Examples 1 to 3 all have high ease of handling. In particular, the wire-equipped stents of Examples 1 and 2 with average tensile loads of at most 0.7 N were found to have higher ease of handling. In Example 1, a PTFE layer is provided on the outer surface of the distal side D1 portion of the pusher wire 12. In Example 2, a hyaluronic acid resin layer is provided on the outer surface of the distal side D1 portion of the pusher wire 12.

Besides the test described above, a sensory test was carried out to evaluate the ease of handling of the wire-equipped stents of Examples 1 to 3 and the comparative example. In the sensory test, 20 testers each pushed and pulled several times the pusher wire of each wire-equipped stent through a catheter having an S shape as in the test describe above. As a result, all testers evaluated the wire-equipped stents of Examples 1 to 3 as having improved ease of handling. On the other hand, none of the testers evaluated the wire-equipped stent of the comparative example as having improved ease of handling.

While embodiments of the present disclosure have been described, it will be understood that the embodiments are not intended to limit the present disclosure and may be altered or modified in various ways, such as those described below, and such modifications will also fall within the technical scope of the present disclosure. It will also be understood that the advantageous effects shown in the embodiments are merely non-limiting examples of the most advantageous effect brought about according to the present disclosure. The embodiments may be combined, as appropriate, with the modified embodiments described below, although such combinations are not described in detail here.

### Modified Embodiments

The wire-equipped stent 10 according to an embodiment of the present invention may include a coiled member between the distal side D1 end of the pusher wire 12 and the stent 11, and the cover layer 13 (see FIG. 1) may be provided on the outer surface of the coiled member. In this embodiment, the coiled member together with the cover layer 13 provides a highly smooth portion. In this embodiment, the cover layer 13 may include a solid layer of a hydrophobic resin or a fluid layer of a hydrophilic resin.

The above embodiment shows an example in which the wire-equipped stent 10 includes the stent 11 of a retrieval type. Alternatively, the connected stent in the wire-equipped stent 10 may be an indwelling stent. In such a case, for example, the outer surface of a deflated balloon may be covered with the radially compressed stent, and the balloon may be inflated at a lesion site to expand the blood vessel and the stent. Thereafter, the balloon may be deflated and then removed together with the pusher wire so that only the stent is indwelled at the lesion site.

The above embodiment shows an example in which the highly smooth portion (e.g., the cover layer 13) has an outer surface with a surface roughness Ra of 0.5 nm or more and less than 2.0 nm. It will be understood that this is a non-limiting example, and alternatively, the outer surface of the highly smooth portion may have an average height Zc of 10 µm to 100 µm according to JIS B 0601.

The above embodiment shows an example in which the cover layer 13 may include a solid layer of a hydrophobic resin or a fluid layer of a hydrophilic resin. It will be understood that this is a non-limiting example, and alternatively, the cover layer 13 may include a solid layer including a hydrophobic material. The solid layer including a hydrophobic material may include, for example, diamond-like carbon (DLC) or the like. Alternatively, the cover layer 13 may include a fluid layer including a hydrophilic material. The fluid layer including a hydrophilic material may include, for example, silicone or the like.

### EXPLANATION OF REFERENCE NUMERALS

1: Catheter-stent system
10: Wire-equipped stent
11: Stent
12: Pusher wire
13: Cover layer
20: Catheter
21: Core material portion
211: Mesh coil
212: Spiral coil

## Claims

1. A wire-equipped stent (10) comprising:
a wire (12);
a stent (11) connected to a distal end portion of the wire (12); **characterized by**
a cover layer (13) provided to cover the outer on a distal side (D1) portion of the wire to increase the smoothness for contact with the inner surface of a catheter (20), wherein the cover layer (13) includes no coiled member provided at or adjacent to a distal portion of the wire (12), wherein a distal side (D1) end portion of the cover layer (13) and a marker (14) which is connected to a proximal side (D2) end portion of the stent (11) are bonded with each other with an adhesive without a gap or with a gap between the distal side (D1) end portion and the marker (14).

2. The wire-equipped stent (10) according to claim 1, wherein the cover layer (13) has an outer surface with a surface roughness Ra of 0.5 nm or more and less than 2.0 nm.

3. The wire-equipped stent (10) according to claim 1, wherein the cover layer (13) comprises a solid layer comprising a hydrophobic material.

4. The wire-equipped stent (10) according to claim 1, wherein the cover layer (13) comprises a fluid layer comprising a hydrophilic material.

5. The wire-equipped stent (10) according to any one of claims 1 to 4, wherein the stent (11) is adapted to be delivered together with the catheter (20) to a region at or downstream of M1 region in a skull and to be used together with the catheter (20) in the skull.

6. The wire-equipped stent (10) according to claim 5, wherein the stent (11) is adapted for use to retrieve a blood clot formed in a cerebral blood vessel in a region at or downstream of the M1 region.

## Patentansprüche

1. Mit Draht ausgestatteter Stent (10), umfassend:
einen Draht (12);
einen mit einem distalen Endabschnitt des Drahts (12) verbundenen Stent (11); **gekennzeichnet durch**
eine Deckschicht (13), die zum Abdecken der Außenseite eines distalen Seitenabschnitts (D1) des Drahts bereitgestellt ist, um die Glätte für den Kontakt mit der Innenfläche eines Katheters (20) zu erhöhen, wobei die Deckschicht (13) kein gewickeltes Element an oder neben einem distalen Abschnitt des Drahts (12) einschließt, wobei ein distaler Seitenendabschnitt (D1) der Deckschicht (13) und eine Markierung (14), die mit einem proximalen Seitenendabschnitt (D2) des Stents (11) verbunden ist, mit einem Klebstoff ohne Spalt oder mit einem Spalt zwischen dem distalen Seitenendabschnitt (D1) und der Markierung (14) aneinander gebondet sind.

2. Mit Draht ausgestatteter Stent (10) nach Anspruch 1, wobei die Deckschicht (13) eine Außenfläche mit einer Oberflächenrauheit Ra von 0,5 nm oder mehr und weniger als 2,0 nm aufweist.

3. Mit Draht ausgestatteter Stent (10) nach Anspruch 1, wobei die Deckschicht (13) eine feste Schicht, umfassend ein hydrophobes Material, umfasst.

4. Mit Draht ausgestatteter Stent (10) nach Anspruch 1, wobei die Deckschicht (13) eine Fluidschicht, umfassend ein hydrophiles Material, umfasst.

5. Mit Draht ausgestatteter Stent (10) nach einem der Ansprüche 1 bis 4, wobei der Stent (11) dazu ausgelegt ist, zusammen mit dem Katheter (20) in eine Region an der M1-Region oder dieser nachgeschaltet in einen Schädel eingeführt und zusammen mit dem Katheter (20) in dem Schädel verwendet zu werden.

6. Mit Draht ausgestatteter Stent (10) nach Anspruch 5, wobei der Stent (11) zur Verwendung, um ein Blutgerinnsel zu entfernen, das sich in einem zerebralen Blutgefäß in einer Region an der M1-Region oder dieser nachgeschaltet gebildet hat, ausgelegt ist.

## Revendications

1. Endoprothèse équipée d'un fil (10) comprenant :
un fil (12) ;
une endoprothèse (11) reliée à une partie distale du fil (12) ; **caractérisée par**
une couche de couverture (13) destinée à recouvrir la partie externe du côté distal (D1) du fil afin d'augmenter la douceur du contact avec la surface interne d'un cathéter (20), dans laquelle la couche de couverture (13) ne comprend pas d'élément enroulé sur ou à côté d'une partie distale du fil (12), dans lequel un côté distal (D1) de la couche de couverture (13) et un marqueur (14) relié à un côté proximal (D2) de l'endoprothèse (11) sont collés l'un à l'autre avec un adhésif sans espace ou avec un espace entre le côté distal (D1) de la couche de couverture et le marqueur (14).

2. Endoprothèse équipée de fils (10) selon la revendication 1, dans laquelle la couche de couverture (13) a une surface extérieure avec une rugosité de surface Ra de 0,5 nm ou plus et moins de 2,0 nm.

3. Endoprothèse équipée d'un fil (10) selon la revendication 1, dans laquelle la couche de couverture (13) comprend une couche solide composée d'un matériau hydrophobe.

4. Endoprothèse équipée d'un fil (10) selon la revendication 1, dans laquelle la couche de couverture (13) comprend une couche fluide composée d'un matériau hydrophile.

5. Endoprothèse équipée d'un fil (10) selon l'une quelconque des revendications 1 à 4, dans laquelle l'endoprothèse (11) est adaptée pour être délivrée avec le cathéter (20) dans une région située au niveau ou en aval de la région M1 d'un crâne et pour être utilisée avec le cathéter (20) dans le crâne.

6. Endoprothèse équipée d'un fil (10) selon la revendication 5, dans laquelle l'endoprothèse (11) est adaptée pour utilisation afin de récupérer un caillot de sang formé dans un vaisseau sanguin cérébral dans une région située au niveau ou en aval de la région M1.
